(19) Europäisches Patentamt
European Patent Office
Office européen des brevets

(11) EP 1 852 119 A1

(12) # EUROPEAN PATENT APPLICATION
published in accordance with Art. 158(3) EPC

(43) Date of publication:
**07.11.2007 Bulletin 2007/45**

(21) Application number: **06713160.7**

(22) Date of filing: **07.02.2006**

(51) Int Cl.:
***A61K 31/538*** *(2006.01)* ***A61P 33/02*** *(2006.01)*
***A61P 33/06*** *(2006.01)* ***C07D 265/38*** *(2006.01)*

(86) International application number:
**PCT/JP2006/302017**

(87) International publication number:
**WO 2006/087935 (24.08.2006 Gazette 2006/34)**

(84) Designated Contracting States:
**CH DE FR GB IT LI**

(30) Priority: **17.02.2005 JP 2005041238**

(71) Applicants:
- **Japan Science and Technology Agency**
**Kawaguchi-shi,**
**Saitama 332-0012 (JP)**
- **TOHOKU UNIVERSITY**
**Aoba-ku**
**Sendai-shi, Miyagi 980-8577 (JP)**
- **FUJIFILM Corporation**
**Minato-ku**
**Tokyo 106-8620 (JP)**

(72) Inventors:
- **IHARA, Masataka**
**i, 9820021 (JP)**
- **TAKASU, Kiyosei**
**Miyagi, 9820036 (JP)**
- **KAWAKAMI, Masayuki**
**ashigara-shi, Kanagawa, 2500193 (JP)**
- **KITAGUCHI, Hiroshi**
**ashigara-shi, Kanagawa, 2500193 (JP)**
- **SATOU, Kouzou**
**ashigara-shi, Kanagawa, 2500193 (JP)**

(74) Representative: **Strehl Schübel-Hopf & Partner**
**Maximilianstrasse 54**
**80538 München (DE)**

(54) **PHARMACEUTICAL COMPOSITION CONTAINING PHENOXAZINIUM COMPOUND AS ACTIVE INGREDIENT**

(57) The purpose of the present invention is therefore to provide a pharmaceutical composition, especially that for the treatment and/or prevention of parasitic infection by protozoa, which has a high therapeutic effect and selective toxicity or a life-prolongation effect for the parasitic infection by protozoa.

The present invention is therefore related to a pharmaceutical composition comprising the compound represented by the following general formula (1) as an active ingredient, especially to those for the treatment and/or prevention of parasitic infection by protozoa.

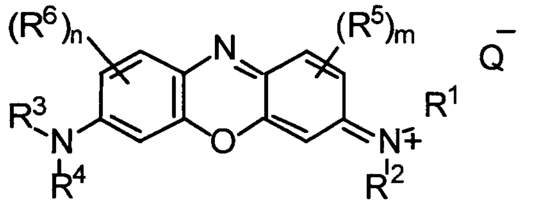

(1)

EP 1 852 119 A1

## Description

Technical Field

The present invention relates to a pharmaceutical composition and novel compounds used as an active ingredient of the pharmaceutical composition. The compounds according to the present invention are useful especially for the treatment and/or prevention of disorders associated with the infection with parasites such as malaria including drug-resistant malaria, leishmaniasis, trypanosomiasis including African sleeping disease and Chagas' disease, toxoplasmosis, and cryptospolidiosis.

Background of the Invention

Parasitic infection diseases by protozoa are frequently observed still now mainly in the tropical and subtropical areas, which include, for example, malaria, leishmaniasis, African trypanosomiasis (African sleeping disease), American trypanosomiasis (Chagas' disease), toxoplasmosis, lymphofilariasis, babesiasis, and cryptospolidiosis. They are classified into those infectious only with human, those infectious with both human and farm or small animals, both of which would cause serious, economic and social damage. Some of the patients infected with the above diseases present such a severe symptom that they will not be able to spend a normal social life, or they will have to be confined to their bed for a long time requiring nursing care. Some of the above diseases may even develop a lethal symptom. However, there is no clinically available vaccine that is effective against the above diseases, and such vaccine is thought to be difficult to develop still in the future.

There is no therapeutic product for some of the above diseases. Some of the therapeutic products for the above diseases have problems that they have caused an appearance and spreading of resistant protozoa and their serious side effects. For example, since Pentostam, the therapeutic agent for leishmaniasis, has antimony atom in its molecule, toxication due to the antimony atom cannot be helped as a side effect involved in the treatment. Melarsoprol, that is used in the initial treatment of African trypanosomiasis has arsenic atom in its molecule, which will cause as a side effect due to arsenic toxication. For the above reasons, it is strongly desired to develop as soon as possible effective therapeutic products that may be administered orally, parenterally and the like

It is already known that /phenoxazine compounds represented by the following formula (2) having a low oxidization level has an inhibiting activity for dehydrofolic acid reductase derived from Toxoplasma gondii that will cause trypanosomiasis (Patent Document 1), suggesting that said compounds may be effective for the parasitic infection diseases. However, it is still unclear that the compounds can also show any therapeutic effect on other parasites than Toxoplasma, living parasites, or parasites in an actually parasitic state in a host, i.e., in a cellular or individual level. Causal relationship between the compounds and their potential effect for the parasitic infection diseases has been completely unclear and impossible to predict.

[Formula 1]

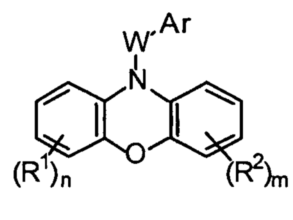

( 2 )

Brilliant Cresyl Blue represented by the following formula (3) is known to show a growth-inhibiting effect for the protozoa of Plasmodium falciparum W2 that will cause malaria under *in vitro* (cell) assay system (Non-Patent Document 1). However, it did not show a high treating effect of malaria under *in vivo* assay using living animals. Accordingly, it is not possible to predict from the disclosure of Non-Patent Document 1 that the phenoxazine compounds having two di-substituted amino groups may have any therapeutic effect for malaria. Furthermore, this document did not mention any possibility of its therapeutic effect for other parasitic infection diseases than malaria at all. And, causal relationship between this compound and its potential effect for the parasitic infection diseases has been completely unclear and impossible to predict.

[Formula 2]

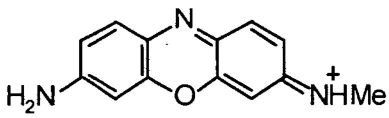

(3)

Non- Patent Document 1 shows the growth-inhibiting effect of the phenoxazine of the formula (3) and the following compounds similar to it for the protozoa of falciparum malaria (Plasmodium falciparum W2 under *in vitro*. EC50 values of these compounds for the above protozoa are summarized in Table 1.

[Formula 3]

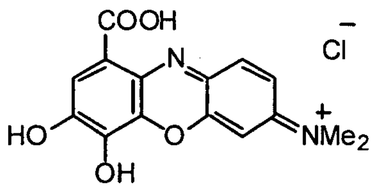

Gallocyanine

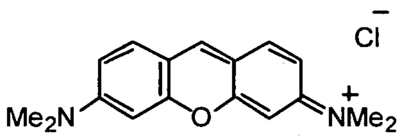

Pyronine Y

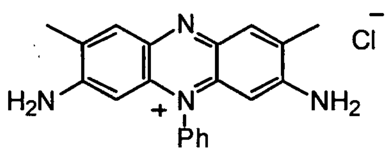

Safranine O

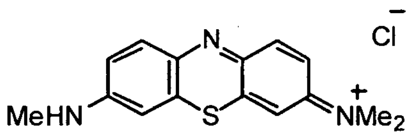

Azure B

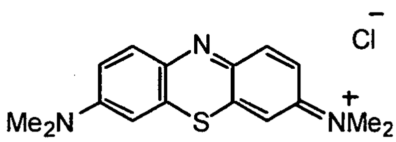

Methylene Blue

[TABLE 1]

| Compound | $EC_{50}$ (nM) (*Plasmodium falciparum W2*) |
|---|---|
| Brilliant Cresyl Blue | 5.52±3.06 |
| Gallocyanine | 674±159 |
| Pyronin Y | 449±201 |
| Safranine O | 34.8±17.2 |
| Azure B | 12.7±7.36 |

(continued)

| Compound | $EC_{50}$ (nM) (*Plasmodium falciparum W2*) |
|---|---|
| Methylene Blue | 3.99±2.31 |

Cure rate (suppression), i.e., a ratio in reduction of the number of erythrocytes infected with malaria in blood was obtained after 4-day successive treatment of mice infected with malaria according to peritoneal administration program of 5mg/kg/day. The results are shown in Table 2. The suppression of 100% means a complete cure.

[TABLE 2]

| Compound | Suppression(%) |
|---|---|
| Brilliant Cresyl Blue | 11.0 |
| Gallocyanine | 15.6 |
| Pyronin Y | 20.6 |
| Safranine O | 14.0 |
| Azure B | 9.1 |
| Methylene Blue | 15.9 |

The above results teach that the compounds disclosed in Non-Patent Document 1 did not show a high growth-inhibiting effect under the *in vivo* assay system, and that their actual therapeutic effect can not be expected. Furthermore, the animals treated with the compounds could obtain life prolongation of only about one or two days compared to non-treated animals. Having a high acute toxicity, they are not suitable to be administered in a large amount and it will be very hard to realize a high curing effect with these compounds.
[Patent Document 1] PCT/US00/01968
[Non-Patent Document 1] Antimicrobial Agents and Chemotherapy, 1995, Vol.39, pp.2671-2677

Summary of the Invention

The purpose of the present invention is therefore to provide a pharmaceutical composition, especially that for the treatment and/or prevention of parasitic infection by protozoa, which has a high therapeutic effect and selective toxicity or a life-prolongation effect for the parasitic infection by protozoa.
The inventors have devoted themselves finally to find that a pharmaceutical composition comprising the compounds represented by the general formula (1) show a high growth-inhibiting effect for the parasitic infection by protozoa even under *in vivo* assay system. The present invention was made based on the above findings.
Thus, the present invention is related to the following aspects.

1. A pharmaceutical composition comprising the compound represented by the following general formula (1) as an active ingredient:

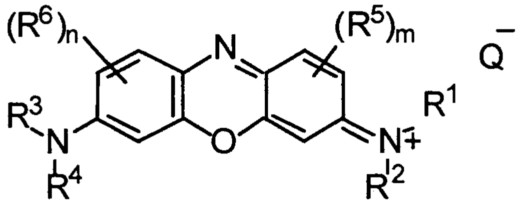

(1)

Wherein R1 and R2 may be the same or different, and independently represent alkyl, aryl, or heterocyclic group, which may optionally have a substituent of hydroxy, alkoxy, halogen, amino, cyano, sulfonic acid, carboxy, ester, amido, or nitro group, or may be condensed together to form a ring;
R3 and R4 may be the same or different, and independently represent alkyl, aryl, or heterocyclic group, which may

optionally have a substituent of hydroxy, alkoxy, halogen, amino, cyano, sulfonic acid, carboxy, ester, amido, or nitro group, or may be condensed together to form a ring;

R5 and R6 may be the same or different, and independently represent halogen, alkyl, aryl, or heterocyclic, hydroxy, alkoxy, acyloxy, amino, cyano, sulfonic acid, carboxy, ester, amido, or nitro group, or may form cycloaliphatic ring, aromatic ring, hetero cycloaliphatic ring, or hetero aromatic ring;

m and n are independently represent an integer of 0 - 3; and

Q is a pharmaceutically acceptable anion.

2. A pharmaceutical composition according to Claim 1 wherein R1, R2, R3 and R4 are independently alkyl group having 1~6 carbon atoms.

3. A pharmaceutical composition according to Claim 1 or 2 wherein Q is halogen ion or perchlorate ion.

4. A pharmaceutical composition according to any one of Claims 1 - 3 wherein R1 and R2, or R3 and R4 are condensed together to form a ring.

5. A pharmaceutical composition according to Claim 4 wherein the ring is piperazine ring or morpholine ring.

6. A pharmaceutical composition comprising any one of the following compounds as an active ingredient:

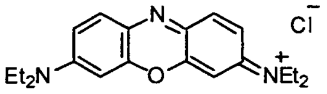
A-1

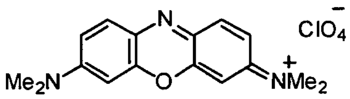
A-2

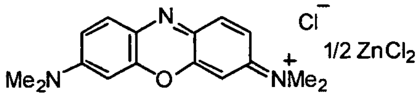
A-3

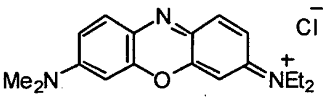
A-4

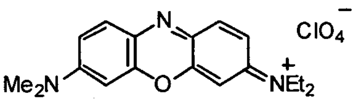
A-5

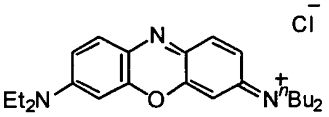
A-6

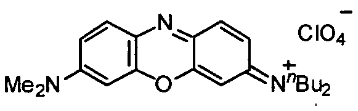
A-7

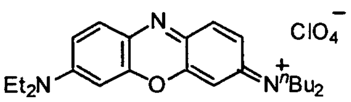
A-8

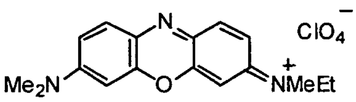
A-9

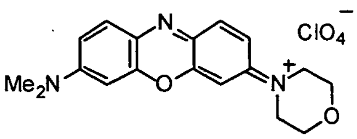
A-10

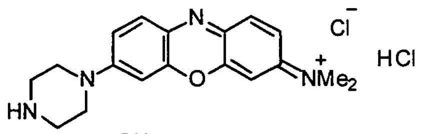
A-11

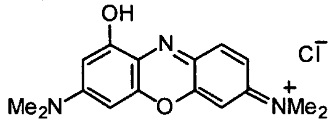
A-12

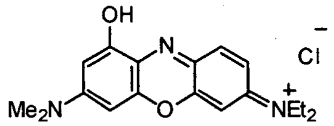
A-13

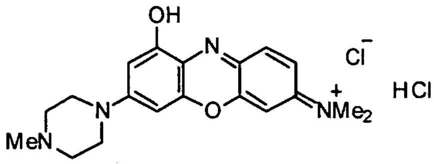
A-14

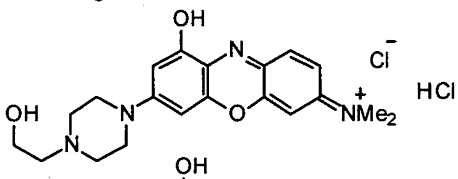
A-15

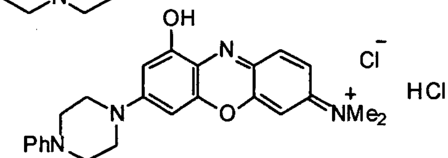
A-16

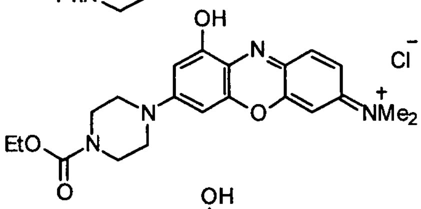
A-17

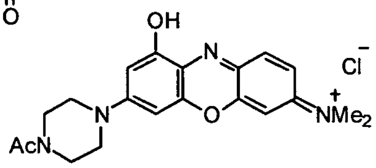
A-18

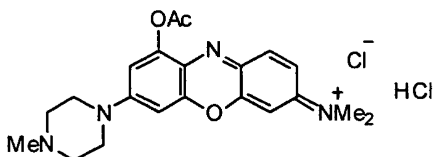
A-19

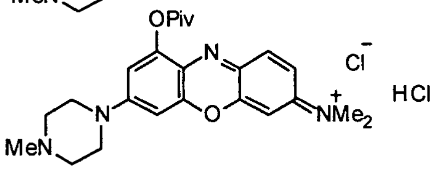
A-20

7. A pharmaceutical composition according to any one of Claims 1 - 6 for the treatment and/or prevention of parasitic infection by protozoa.

8. A pharmaceutical composition according to Claim 7 wherein the parasitic infection is malaria, leishmaniasis, African sleeping disease, Chagas' disease, toxoplasmosis, lymphofilariasis, babesiasis, or coccidiosis.

9. A pharmaceutical composition according to Claim 8 wherein the parasitic infection is malaria, leishmaniasis, African sleeping disease or Chagas' disease.

10. A pharmaceutical composition according to Claim 9 wherein the parasitic infection is malaria.

11. A pharmaceutical composition according to any one of Claims 1 - 10 for the treatment and/or prevention of the parasitic infection by protozoa, comprising the compound represented by the general formula (1) in an amount of 1 mg ~ 10,000 mg.

12. A pharmaceutical composition according to any one of Claims 1 - 11 for the treatment and/or prevention of the parasitic infection by protozoa, wherein said composition is in a form of liquid, tablet, or pill or colloid.

13. A phenoxazinium compound represented by any one of the structures named as A-8, A-9, and A-11.

The compounds comprised as the active ingredient in the pharmaceutical composition according to the present invention will show the growth-inhibiting effect even by its administration in a small amount especially for the parasitic infection by protozoa. They would not harm mammalian cells even when they were administered with a higher dose than that showing the growth-inhibiting effect for the protozoa of parasites. Thus, they have a high selective toxicity. It was also confirmed by *in vivo* assay test that the above compounds could inhibit the growth of the protozoa of parasites without showing any side effects such as an acute toxicity, and show therapeutic effect and significant life prolongation effect. Furthermore, since the compounds according to the present invention do not contain poisonous atoms such as antimony and arsenic, they have no risk to cause any side effects.

Best Mode for Carrying Out the Invention

The pharmaceutical composition according to the present invention will be more specifically explained below.

Various compounds were examined with respect to their growth-inhibiting effects for the protozoa of parasites that are an etiologic factor, and assayed with respect to their cytotoxicity for mammalian cells. They were administered in various amounts and forms into a mouse infected with malaria as a host model in order to assay their therapeutic effects. The above *in vivo* assay system was used to search a compound that would show 50 % or more increase in the growth-inhibiting effect for the protozoa of malaria at a dose of 5~10 mg/kg in comparison with a non-treated case.

As a result, the compounds represented by the general formula (1) were found to show the above effects. These compounds will be explained more in detail below.

The alkyl groups of R1 - R4 in the general formula (1) have preferably 1~12 carbon atoms, more preferably 1~6 carbon atoms, and may be linear, branched or cyclic ones. Examples of the alkyl groups include methyl, ethyl and butyl. The alkyl groups may have a substituent.

The aryl groups of R1 - R4 in the general formula (1) have preferably 5~15 carbon atoms, more preferably 6~10 carbon atoms.

The heterocyclic groups of R1 - R4 in the general formula (1) are preferably a 5~8-membered ring, more preferably a 5 or 6-membered ring. The hetero atom includes nitrogen, oxygen, sulfur, selenium, tellurium and phosphorous. Among them, nitrogen, oxygen, sulfur and selenium are preferred. The examples of the heterocyclic groups include pyrrole, furan, piperidine, morpholine, piperazine, pyridine, and pyrrolidine, which may have a substituent.

R1 and R2, or R3 and R4 may be condensed together to form a 3~12 membered, preferably 5~7 membered saturated or unsaturated ring. The examples of the saturated or unsaturated ring include a heterocyclic ring such as piperidine, piperazine, morpholine, azepine, pyrrole, and tetrahydro pyridine.

The examples of the cycloaliphatic ring, aromatic ring, hetero cycloaliphatic ring, or hetero aromatic ring that may be formed by the condensation of R5 and R6 include cyclohexene, cyclopentene, benzene, naphthalene, dihydropyrrole, tetrahydropyridine, pyrrole, furan, pyridine and indole rings.

Preferable examples of the above substituent include an alkyl group such as methyl, ethyl, propyl and isopropyl groups; an aromatic group such as phenyl and napthyl; amino; dialkylamino; hydroxy; alkoxy; acyloxy; carboxy; alkoxycarbonyl; aminocarbonyl; nitrile; sulfonic acid; nitro; chloro; fluoro; and bromo groups.

"Q" is necessary for charge equilibrium in the compounds of the general formula (1). The term "pharmaceutically acceptable anion" with respect to "Q" means an ion that does not show any toxicity when administered to a recipient and can dissolve the above compound in an aqueous system.

Preferable examples of "Q" include a halogen ion such as chlorine, bromo and iodine ion; a sulfonate ion such as aliphatic and aromatic sulfonate ion including methanesulfonate, trifluoromethanesulfonate, p-tolune sulfonate, naphthane sulfonate, 2-hydroxyethanesulfonate ions; a sulfamate ion such as cyclohexane sulfamate ion; a sulfate ion such as methylsulfate and ethylsulfate ions; hydrogen sulfate ion; borate ion; alkyl- and dialkyl-phosphate ions such as diethylphosphate ion and methyl hydrogen phosphate ion; a pyrophosphate ion such as trimethylpyrophosphate ion; a carboxlate ion, carboxy and hydroxy groups of which may be often replaced; a carbonate ion; a hydrogen carbonate ion; perchlorate ion; and a hydroxide ion. Especially preferable examples of "Q" include the chlorine ion, acetate ion, propionate ion, valerate ion, citrate ion, maleate ion, fumarate ion, lactate ion, succinate ion, tartrate ion, benzoate ion, perchlorate ion, and hydroxide ion.

Typical examples of the above compounds may be described as follows. However, it should not be construed

that the compounds used in the present invention will be limited to the above ones. The compounds named as "A-8", "A-9" and "A-11" are novel ones, and the present invention relates also to these compounds per se.

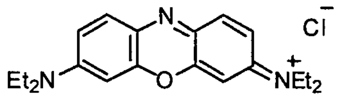
A-1

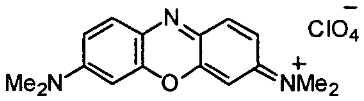
A-2

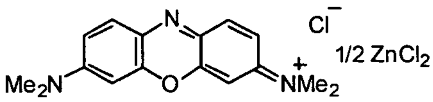
A-3

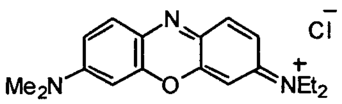
A-4

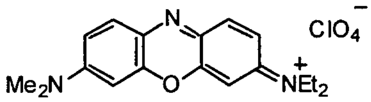
A-5

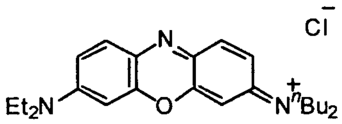
A-6

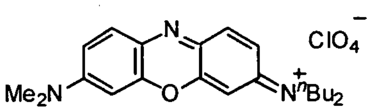
A-7

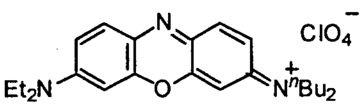
A-8

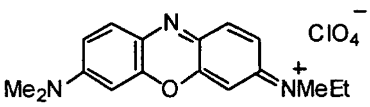
A-9

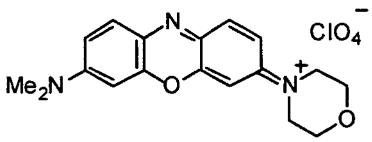
A-10

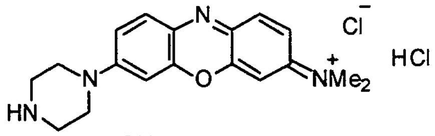
A-11

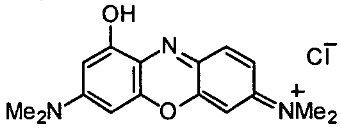
A-12

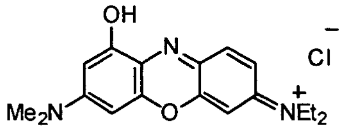
A-13

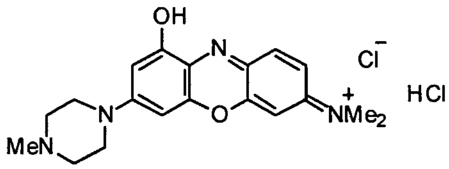
A-14

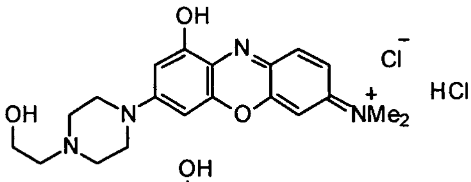
A-15

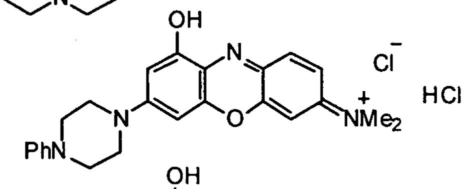
A-16

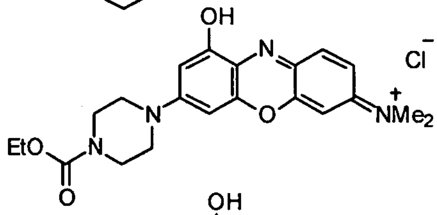
A-17

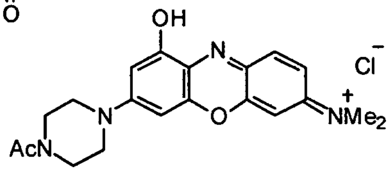
A-18

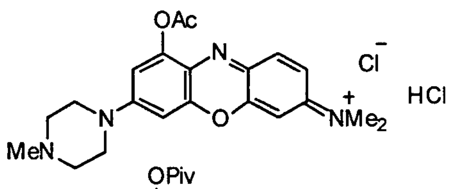
A-19

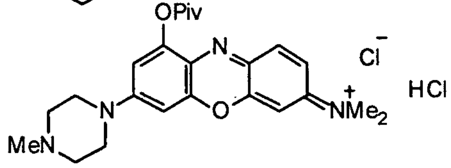
A-20

The phenoxazinium compounds represented by the general formula (1) according to the present invention may

be easily synthesized from known starting materials with reference to known techniques such as those disclosed in Non-Patent Document 1 and Journal of American Chemical Society, 1952, Vol.74, p.578-584 by M.L. Crossley et al., and in European Journal of Organic Chemistry, 1999, Vol.4, p.923-930 by K. Andreas et al. The whole disclosures of these documents are incorporated into the present specification by reference.

The pharmaceutical composition comprising the compound represented by the following general formula (1) is useful especially for the treatment and/or prevention of various types of disorders associated with the infection with parasitic protozoa as such malaria, African trypanosomiasis (African sleeping disease), American trypanosomiasis (Chagas' disease), leishmaniasis, babesiasis, lymphofilariasis, toxoplasmosis (opportunistic infectious diseases such as AIDS), and cryptospolidiosis (tropical diarrhea)

One or more kinds of the compounds of the general formula (1) may be comprised in the pharmaceutical composition as the active ingredient according to the present invention, and may further be used optionally in combination with other therapeutic agents such as those known for those skilled in the art for the parasitic infection diseases. Preferable examples of the agents used for the parasitic infection diseases include Chloroquine, Mefloquine, Altemisinin, Atovaquone and Pyrimethamine (for malaria); Suramin, Pentamidine, Melarsoprol, and Ascofuranone (for African sleeping disease); Benznnidazole for Chagas' disease; Pentostam, Amphotericin B, Miltefosine, Fluconazole for leishmaniasis.

Pharmaceutical carries or diluents that may be used in combination with the compounds of the general formula (1) according to the present invention include sodium chloride; magnesium chloride; zinc chloride; glucose; sucrose; lactose; ethylalcohol; glycerine; mannnitol; sorbitol; pentaerythritol; diethylene glycol, propylene glycol, dipropylene glycol, polyethylene glycol 400 and other polyethylene glycols; mono-, di-, tri-glyceride of an aliphatic acid such as glyceride with trilauric acid and glyceride with distearic acid; pectin; starch; alginic acid; xylose; talc; lycopodium; oil and fat such as olive oil, peanut oil, castor oil, corn oil, safflower oil, wheat germ oil, sesami oil, cotton seed oil, sunflower oil, and oleum morrhuae; gelatin; lecithin, silica; cellulose; cellulose derivatives such as methylhydroxypropyle cellulose, methylcellulose and hydroxyethyl cellulose; salts of aliphatic acids having 12-22 carbon atoms such as calcium stearate, calciium laurate, magnesium oleate, calcium palmitate, calcium behenate and magnesium searate; cyclodextrins such as α-cyclodextrin, β-cyclodextrin, γ-cyclodextrin, hydroxyethyl- β-cyclodextrin, hydroxypropyl- β-cyclodextrin, dihydroxypropyl- β-cyclodextrin, carboxymethylethyl- β-cyclodextrin, Cycloawaodorin, and dimethyl- β-cyclodextrin; emulsifier such as esters of a saturate or unsaturated aliphatic acid having, for example, 2-22, preferably 10-18 carbon atoms, and an aliphatic monoalcohol or polyalcohol having 1-20 carbon atoms such as glycol, glycerine, diehtylen glycol, pentaerythrytol, ethylalcohol, butylalcohol, and octadecylalcohol; and silicon such as dimethyl polysiloxane. Furthermore, the pharmaceutical composition of the present invention may further contain any additional carries that have been conventionally used in the pharmaceutical composition and known for those skilled in the art.

Pharmaceutically effective amount and administration route or means of the compounds according to the present invention may be optionally selected by those skilled in the art depending on a kind of the parasites causing the diseases, a location of parasitic part, severity of the diseases, therapeutic strategy, and the age, weight, sex, general health conditions and racial (genetic) background of a patient. Generally, the compounds may be administered in an amount of 1 mg ~ 10,000 mg/day/70 kg of a body weight, more generally 50 mg ~ 2,000 mg/day/70 kg of a body weight.

The pharmaceutical composition according to the present invention may be prepared into any kind of formulation known for those skilled in the art depending on the administration route, means and the like. For example, the pharmaceutical composition with the above carriers or diluents may be administered in a form of liquid, tablet or colloid. The liquid formulation may be injected intravenously, intraperitoneally, or subcutaneously as a dissolved form in 5% glucose aqueous solution or with the above carries or diluents. The tablet formulation may be orally administered, and the colloid formulation may be applied on skin. The pharmaceutical composition may comprise the compounds in an appropriate amount depending on its purpose and form, subject to be administered and the like, for example, normally about 1 mg ~ 10,000 mg, preferably about 10 mg ~ 3,000 mg.

The compounds represented by the general formula (1) and advantages of the pharmaceutical composition comprising them as the active ingredient will be illustrated below with reference to the examples. The scope of the present invention, however, is not to limited to them. The compund named as "A-1 (60% purity)" was purchased from MP Biomedical Product, Co. and used as such without any further purification. The other compounds used in the examples had purity of 95% or more, as ensured by 1 H-NMR and elementary analysis.

## Example 1

### Synthesis of 3-dibutylamino-7-diethylamino phenoxazinium perchlorate (Compound A-8)

A mixture of 3-dibutylaminophenol (1.0mL, 4.43mmol) and N,N-diethyl-4-nitrosoaniline (790mg, 4.43mmol) was suspended in ethanol (55mL) at a room temperature, and 60% aqueous solution of perchloric acid (0.5mL) was added by dropping to the suspension. The resulting mixture was refluxed with heating for 3 hours and cooled to a room temperature. It was then concentrated under reduced pressure to half an initial volume of its solvent, and cooled to 0°C.

The resulting precipitate was removed by filtration and filtrate was concentrated. The concentrated crude material was purified by means of silica chromatography (eluate: chloroform:ethyl acetate=9:1) to give a crude compound. The resulting crude compound was dissolved into methanol and cooled to 0°C, to which added few drops of diethylether for crystallization. The resulting dark blue crystal was filtered to give -dibutylamino-7-diethylamino phenoxazinium perchlorate (33.3mg, isolation yield of 2%).
1 H-NMR(400MHz,CD30D)
δ:7.77(d,2H,J=9.8Hz),7.38(dd, 1H,J=9.8,2.6Hz),7.35(dd, 1H,J=9.8,2.6Hz),3.77(q, 4H,J=7.1 Hz),3.70(q,4H,J=7.8Hz), 1.74(m,4H),1.46(m,4H),1.35(t,6H,J=7.1 Hz),1. 02(t,6H,J=7.5Hz). FAB-MS 380.

Example 2

Synthesis of 3-ethylmethylamino-7-dimethylamino phenoxazinium perchlorate (Compound A-9)

A mixture of 3-methlethylaminophenol (300mg, 1.98mmol) and N,N-dimethyl-4-nitrosoaniline (298mg, 1.98mmol) was suspended in ethanol (15mL) at a room temperature, and 70% aqueous solution of perchloric acid (0.5mL) was added by dropping to the suspension. The resulting mixture was refluxed with heating for 6 hours, cooled to a room temperature and distilled under reduced pressure to remove the solvent. The resulting crude material was purified by means of silica gel chromatography (eluate: chloroform:ethyl acetate=9:1) to give a crude compound (216mg, crude yield of 27%). The resulting crude compound was dissolved into methanol and cooled to 0°C for crystallization. The resulting dark blue crystal was filtered to give 3-ethylmethylamino-7-dimethylamino phenoxazinium perchlorate (13.3 mg, isolation yield of 2 %).
1H-NMR(400MHz,CDCl3)
δ:7.80(s,1H),7.78(s,1H),7.41 (dd,1H,J=6.8,2.8Hz),7.39(dd,1H,J=6.8,2.8Hz),6.96( d,1H,J=2.8Hz),6.94(d,1H,J=2.8Hz), 3.81 (q,4H,J=7.2Hz),3.41(s,6H),3.38(s,3H),1. 34(t,3H,J=7.2Hz). FAB-MS 282.

Example 3

Synthesis of 3-dimethylamino-7-(1-piperazino) phenoxazinium chloride monohydrochloride salt (CompoundA-11)

A mixture of 3-(1-piperazino) phenol (165mg, 0.92mmol) and N,N-dimethyl-4-nitrosoaniline (139mg, 0.92mmol) was suspended in ethanol (50mL) at a room temperature, and 70% aqueous solution of perchloric acid (0.5mL) was added by dropping to the suspension. The resulting mixture was refluxed with heating for 6 hours, cooled to a room temperature and distilled under reduced pressure to remove the solvent. The resulting crude material was purified by means of silica gel chromatography (eluate: chloroform:ethyl acetate=9:1) to give a crude compound (65mg). The resulting crude compound was dissolved into methanol, mixed with ion-exchange resin Amberlyte IRA-400 (C1) and left to stand for 2 hours at a room temperature. The mixture was then filtered, and the resin was further washed with methanol. The collected filtrate was concentrated under reduced pressure for crystallization. The resulting dark blue crystal was filtered to give 3-dimethylamino-7-dimethylamino7-(1-piperazino) phenoxazinium chloride mono- hydrochloride salt (49.2 mg, isolation yield of 14 %).
1 H-NMR(400MHz,CD30D)
δ:7.91 (d,1 H,J=9.3Hz),7.84(d, 1H,J=9.6Hz),7.59(dd,1 H,J=9.6,2.7Hz),7.49(dd,1 H, J=9.3,2.7Hz),7.22(d,1 H,J=2.7Hz), 7.07(d, 1H,J=2.7Hz),4.04(t,4H,J=5.3Hz), 3.52(s,6H),3.46(t,4H,J=5.3Hz). FAB-MS 309.
The other compounds (A-1~A-20) according to the present invention were synthesized in a similar manner.

Example 4

Culture of the protozoa of chloroquine-resistant protozoa of falciparum malaria

Plasmodium Falciparum K1 strain was used as a chloroquine-resistant protozoa of falciparum malaria. RMI-1640 sterilized by filtration was supplemented with human serum of a final concentration of 5% to be used as a culture medium. The malaria protozoa were cultured under $O_2$ concentration of 3%, $CO_2$ concentration of 4%, $N_2$ concentration of 93% at 37°C.

Screening assay *in vitro* of growth-inhibiting effect for chloroquine-resistant protozoa of falciparum malaria

A test sample was prepared by dissolving the test compounds according to the present invention and a positive control drug (chloroquine) in DMSO to a predetermined concentration. The erythrocytes infected with the cultured malaria protozoa were collected by centrifugation and so diluted with non-infected erythrocytes to have an initial infection ratio

of 0.15%. The hematocrit value was then 2.5%. Culture mixture of the above malaria-infection culture medium (200 μL) was placed into each well of 96-well culture plate, and the test sample comprising the predetermined amount of the test compound, and DMSO without the test compound were added to the wells in duplicate.

After culture for 48 hours at 37°C, hypoxanthine labeled with radioactive tritium ($^3H$) of 0.5μCi was added to each well. After the culture in the same conditions for further 24 hours, the sample was taken on a glass fiber filter and washed with distilled water. Intensity of radiation was measured by means of β-plate liquid scintillation counter (Wallac Co.), and an infection ratio of the protozoa of malaria was calculated with respect to a group treated with the test sample and the control. A growth-inhibiting ratio was then calculated from the resulting infection ratio in accordance with the following equation to determine 50% growth-inhibiting concentration ($EC_{50}$).

$$\text{Growth-inhibiting ratio (\%)} = [1-(b-a) / (c-a)] \times 100$$

a: Initial infection ratio;
b: Infection ratio of the test sample group; and
c: Infection ratio of the control group

*In vitro* growth-inhibiting assay of rat L6 cells

Rat L6 cell (rat skeletal myoblast cell) was cultured in RPMI-1640 culture medium supplemented with 1% of L-glutamine (200mM) and fetal calf serum (10%) under 5% $CO_2$ concentration at 37°C. A test sample was prepared by dissolving the test compounds according to the present invention and a positive control drug in DMSO to a predetermined concentration. After pre-culture had been finished, the culture medium containing the cells in a logarithmic phase was placed into each well of 96-well culture plate, and the test sample comprising the predetermined amount of the test compound, and DMSO without the test compound were added to the wells in duplicate.

After incubation of the culture plate for 72 hours in an incubator, a growth-inhibiting activity was assayed as follows. Alamar Blue aqueous solution (10 μL) was added to each well followed by a further two-hour culture. The culture plate was then attached to a fluorescence micro titer plate reader (Spectramax Gemeni XS; U.S. Molecular Device Co.), and the intensity of fluorescence at 588nm was detected with an excitation wave length of 536nm. A residual ratio of L6 cells was calculated with respect to a group treated with the test sample and a control group. A growth-inhibiting ratio for L6 cells was then calculated from the resulting residual ratio in accordance with the following equation to determine 50% growth-inhibiting concentration ($EC_{50}$).

$$\text{Growth-inhibiting ratio (\%)} = [1-(C-A)/(B-A)] \times 100$$

A: Initial number of cells;
B: Number of the cells in the control after 3 days; and
C: Number of the cells treated with the test sample after 3 days

Determination of pharmaceutical effect for chloroquine-resistant malaria

Anti-malaria effect of the samples was assessed by their EC 50 values for the chloroquine-resistant protozoa of falciparum malaria and the rat L6 cell. A chemotherapy coefficient, which is used as an index for the selective toxicity for the chloroquine-resistant protozoa of falciparum malaria was calculated by the following equation, and the pharmaceutical effect was determined.

Chemotherapy coefficient = ($EC_{50}$ value of the test sample for rat L6 cell) /($EC_{50}$ value of the test sample for the chloroquine-resistant protozoa of falciparum malaria)

The values of $EC_{50}$ for the rat L6 cell and the chloroquine-resistant protozoa of falciparum malaria, and the selective toxicity of the compounds according to the present invention and the positive control drug are shown in TABLE 3. These results demonstrated that the compounds according to the present invention did show a remarkably excellent growth-inhibiting effect and a highly selective toxicity for the chloroquine-resistant protozoa of falciparum malaria.

[TABLE 3]

| Compound | 50% growth-inhibiting concentration (n M) | | Selective toxicity |
|---|---|---|---|
| | *P. falciparum* K1 | cytotoxicity L6 | |
| A-1 (60% purity) | 4.6 | 3950 | 860 |
| A-1 (>95% purity) | 2.1 | 20500 | 9600 |
| A-3 | 15.6 | 8870 | 570 |
| A-4 | 2.1 | 4690 | 1200 |
| A-6 | 2.4 | 839 | 350 |
| A-7 | 1.5 | 409 | 270 |
| A-11 | 27.5 | 63800 | 2000 |
| A-14 | 196 | 86400 | 440 |
| A-15 | 191 | 74600 | 390 |
| chloroquine | 150 | - | - |

Example 5

*In vivo* treatment test by intraperitoneal administration into mouse infected with malaria

This test was carried out in accordance with 4-day suppressive test that is a general *in vivo* test for the activity of an anti-malaria compound.

Rodent malaria protozoa (Plasmodium berghei NK65 strain) were used in this test. Infected blood was prepared by infection with intraperitoneal administration into ICR male mouse (5 weeks age; SPF) and passage culture (subculture) thereafter. Blood was drawn from the vein of a tail of the moue infected with malaria, and its infection ratio was determined. After it was confirmed that the infection ratio had been increased up to an appropriated level (10~20%), the blood infected with malaria was drawn from the heart of the mouse. The infection ratio and number (cells/mL) of erythrocytes were determined. An uninfected mouse (ICR male, 5 weeks age) was then infected with an injection of the infected erythrocytes so diluted with PBS as to comprise the protozoa of $1.0 \times 10^{-4}$ per dose (0.2 mL) into the vein of its tail.

A test sample was prepared by dissolving the test compounds according to the present invention into physiologic saline (Otsuka Pharmaceuticals Industries) to a predetermined concentration. In the case where the compound was insoluble in water, it was dissolved into DMSO or Tween 20 to prepare the test sample. A control group (no drug-administration group) was treated only with physiologic saline. Weight of the mouse was determined so that a dose would comprise 5.0 mg per 1 kg of its weight.

One group consisted of five mice. The intraperitoneal administration of the test sample started after two hours from the infection with malaria and continued for successive 4 days with a 24-hour interval. After 24 hours from the last administration, blood was drawn from the tail of the infected mouse to prepare a thin-layer smear, and the number of the erythrocytes infected with the malaria protozoa under a microscope with respect to the control group and the test sample groups. Parasitemia, i.e., the infection ratio with the malaria protozoa, was obtained by calculating an average value of the medium three mice except the two mice showing a maximum or minimum inhibition level. A cure rate (suppression) was then calculated from the resulting parasitemia in accordance with the following equation.

$$\text{Cure rate:suppression (\%)} = (b-a) / b \times 100$$

a: Infection ratio of the test sample group ; and
b: Infection ratio of the control group.

Mice were observed with respect to change in their weight and conditions such as gloss of hair in order to assess side effects such as acute toxicity due to the administration of the drug. The cure rate (%) of the mice infected with malaria after the treatment for 4 days (5mg/kg/day, through the intraperitoneal administration) were shown in TABLE 4.

[TABLE 4]

| Compound | Cure rate (%) |
|---|---|
| A-1 (60% purity) | 46.3 |
| A-1 (>95% purity) | 79.2 |
| A-2 | 45.9 |
| A-3 | 17.1 |
| A-4 | 85.3 |
| A-5 | 77.0 |
| A-6 | 83.1 |
| A-7 | 90.3 |
| A-9 | 71.7 |
| A-11 | 54.8 |
| A-14 | 17.4 |
| A-17 | 38.6 |
| A-19 | 21.4 |
| Non-treated | 0 |

No reduction in weight or change of the conditions of mice was observed, which might be attributed to the side effects due to the intraperitoneal administration (5mg/kg). All the tested compounds according to the present invention showed a significant life-elongation effect when compared with the no drug-administration group. They also showed a remarkably higher cure rate than that shown by the known compounds disclosed in Non-Patent Document 1.

Furthermore, the cure rate was assessed through the intraperitoneal administration of the compounds A-1 (60 % purity), A-4 and A-11 in various amounts of their doses in the above 4-day suppression test. Mice were observed with respect to change in their weight and conditions such as gloss of hair in order to assess side effects such as acute toxicity due to the administration of the drug. The cure rate (%) of the mice infected with malaria after the treatment for 4 days (2.5-20 mg/kg/day, or 5.0-30 mg/kg/day through the intraperitoneal administration) were shown in TABLE 5.

[TABLE 5]

| Compound | Administration amount (mg/kg/day) | Cure rate (%) |
|---|---|---|
| A-1 (60% purity) | 2.5 | 30.5 |
| | 5.0 | 46.3 |
| | 10 | 82.4 |
| | 20 | 95.8 |
| A-4 | 2.5 | 58.7 |
| | 5.0 | 85.3 |
| | 10 | 99.9 |
| | 20 | >99.99 |
| A-11 | 5.0 | 54.8 |
| | 10 | 78.5 |
| | 20 | 98.1 |
| | 30 | >99.99 |

No reduction in weight or change of the conditions of mice, which might be attributed to the side effects, was observed in all of the doses except the cases of 20 mg/kg administration of A-1 and 30 mg/kg administration of A-11.

However, the increase of the weight after the completion of the drug administration was observed also with respect to the above two cases. All of the treatments showed a significant life-elongation effect.

Example 6

*In vivo* treatment test by oral administration into mouse infected with malaria

The cure rate (%) of the mice after 5 days from the infection with malaria and the treatment with A-1 (60% purity) according to Example 5 (25-100mg/kg/day, through the oral administration) were shown in TABLE 6. The oral administration, starting after two hours from the infection with malaria, consisted of four times with a 24-hour interval, 12 times with 8-hour interval or singe dose.

The cure rate (%) of the mice after 5 days from the infection with malaria and the treatment with A-1 (100mg/kg/day, through the oral administration of four times with a 24-hour interval or singe dose) were shown in TABLE 7.

[TABLE 6]

| Administration program | Administration amount | Cure rate (%) |
|---|---|---|
| Four times with a 24-hour interval | 25mg/kg/dose | 82.3 |
| | 50mg/kg/dose | 98.6 |
| | 90mg/kg/dose | >99.99 |
| | 100mg/kg/dose | >99.99* |
| 12 times with 8-hour interval | 15mg/kg/dose | >99.99 |
| Single administration | 100mg/kg/dose | >99.99 |
| * One of five mice was died due to technical error at the time of oral administration of the drug | | |

No reduction in weight or change of the conditions of mice, which might be attributed to the side effects, was observed in all of the dose programs. All of the treatments showed a significant life-elongation effect. Especially, in the program where the dose of 100mg/kg was administered four times with a 24-hour interval, three mice of the four mice in the group (one mouse was died on the day 21) were still alive after 30 days. Blood was drawn from these three surviving mice after 36 days in order to observe the presence of the protozoa of malaria in their erythrocytes. No protozoa of malaria was shown to be present in the blood.

[TABLE 7]

| Administration program | Administration amount | Cure rate (%) |
|---|---|---|
| Four times with 24 -hour interval | 100mg/kg/dose | >99.99 |
| Single administration | 100mg/kg/dose | 78.3 |

No reduction in weight or change of the conditions of mice, which might be attributed to the side effects, was observed in all of the dose programs. All of the treatments showed a significant life-elongation effect.

Example 7

Culture of the protozoa of African trypanosomiasis

This test was done using a trypomastigote of the protozoa of Trypanosoma brucei rhodensiense (STIB 900 strain) ranging in blood stream. MEM medium sterilized by filtration was supplemented with 25 mM of N-2-hydroxyethylpiperazine -2-ethanesulfonic acid (HEPES), 1g/L of glucose, 1% of MEM non-essential amino acids, 0.2mM of 2-mercaptoethanol, 2mM of sodium pyruvate, 1mM of hypoxathine and 15% of heat-treated horse serum. The protozoa were cultured under $CO_2$ concentration of 5%, at 37°C.

*In vitro* screening assay of growth-inhibiting effect for the protozoa of African trypanosomiasis

A test sample was prepared by dissolving the test compounds according to the present invention and a positive control drug (Melarsoprol) in DMSO to a predetermined concentration. Culture medium containing the protozoa of 8 x $10^3$, and the test sample comprising the predetermined amount of the test compound, and DMSO without the test compound were added into each well of 96-well culture plate to a final volume of 100 μL in duplicate.

After incubation of the culture plate for 72 hours in an incubator, a growth-inhibiting activity was assayed as follows. Alamar Blue aqueous solution (10 μL) was added to each well followed by a further two-hour culture. The culture plate was then attached to a fluorescence micro titer plate reader (Spectramax Gemeni XS; U.S. Molecular Device Co.), the intensity of fluorescence at 588nm was detected with excitation wave length of 536nm to calculate an infection ratio of the protozoa with respect to a group treated with the test sample and a control group. A growth-inhibiting ratio was then calculated from the resulting infection ratio in accordance with the following equation to determine 50% growth-inhibiting concentration ($EC_{50}$).

$$\text{Growth-inhibiting ratio (\%)} = [1-(b-a) / (c-a)] \times 100$$

a: Initial infection ratio;
b: Infection ratio of the test sample group; and
c: Infection ratio of the control group.

Determination of pharmaceutical effect for African trypanosomiasis

A selective toxicity coefficient, which is used as an index for the selective toxicity for the protozoa of African trypanosomiasis, was calculated by the following equation, and the pharmaceutical effect was determined.

Selective toxicity coefficient = ($EC_{50}$ value of the test sample for rat L6 cell) /($EC_{50}$ value of the test sample for the protozoa of African trypanosomiasis)

The values of $EC_{50}$ for rat L6 cell and the protozoa of African trypanosomiasis, and the selective toxicity of the compounds according to the present invention and the positive control drug are shown in TABLE 8. These results demonstrated that the compounds according to the present invention did show a lower toxicity for the rat L6 sell than Melarsoprol known as a drug against African trypanosomiasis.

[TABLE 8]

| Compound | 50% growth-inhibiting concentration (nM) | | Selective toxicity |
|---|---|---|---|
| | *Trypanosoma brucei rhod.* | cytotoxicity L6 | |
| A-1 (60% purity) | 181 | 3950 | 22 |
| A-3 | 565 | 8870 | 16 |
| A-4 | 497 | 4690 | 9.4 |
| A-6 | 67.2 | 839 | 12 |
| A-7 | 113 | 409 | 3.6 |
| Melarsoprol | 6 | 7800 | 1300 . |

Example 8

Culture of the protozoa of American trypanosomiasis

The test was done using a trypomastigote and an amastigote of the protozoa of Trypanosoma cruzi (Tulahuen C2C4 strain) present in the infected rat L6 cells. The L6 cells were cultured in RPMI-1640 medium supplemented with 1% of L-glutamate (200mM) and 10% of fetal calf serum under $CO_2$ concentration of 5%, at 37°C.

*In vitro* screening assay of growth-inhibiting effect for the protozoa of American trypanosomiasis

A test sample was prepared by dissolving the test compounds according to the present invention and a positive control drug (benznidazole) in DMSO to a predetermined concentration. Culture medium containing the protozoa of 5 x $10^3$ was added into each well of 96-well culture plate and cultured for 48 hours. After the medium was exchanged, the

test sample comprising the predetermined amount of the test compound, and DMSO without the test compound were added into each well in duplicate.

After incubation of the culture plate for 96 hours in an incubator, agrowth-inhibiting activity was assayed as follows. CPRG/Nonidet (50 $\mu$L) was added to each well followed by a further 2~6-hour culture. The culture plate was then attached to an absorption micro titer plate reader, the absorbance at 540nm was detected to calculate an infection ratio of the protozoa with respect to a group treated with the test sample and a control group. A growth-inhibiting ratio was then calculated from the resulting infection ratio in accordance with the following equation to determine 50% growth-inhibiting concentration ($EC_{50}$).

$$\text{Growth-inhibiting ratio (\%)} = [1-(b-a) / (c-a)] \times 100$$

a: Initial infection ratio;
b: Infection ratio of the test sample group; and
c: Infection ratio of the control group.

<u>Determination of pharmaceutical effect for American trypanosomiasis</u>

A selective toxicity coefficient, which is used as an index for the selective toxicity for the protozoa of American trypanosomiasis, was calculated by the following equation, and the pharmaceutical effect was determined.

A selective toxicity coefficient = ($EC_{50}$ value of the test sample for rat L6 cell) /($EC_{50}$ value of the test sample for the protozoa of American trypanosomiasis)

The values of $EC_{50}$ for rat L6 cell and the protozoa of American trypanosomiasis, and the selective toxicity of the compounds according to the present invention and the positive control drug are shown in TABLE 9. These results demonstrated that the compounds according to the present invention did remarkably show a more excellent growth-inhibiting effect and a more highly selective toxicity than benznidazole known as a drug against American trypanosomiasis.

[TABLE 9]

| Compound | 50% growth-inhibiting concentration (nM) | | Selective toxicity |
|---|---|---|---|
| | *Trypanosoma cruzi* | cytotoxicity L6 | |
| A-1 (60% purity) | 149 | 3950 | 27 |
| A-1 (>95% purity) | 15 | 20500 | 1400 |
| A-3 | 457 | 8870 | 19 |
| A-4 | 211 | 4690 | 22 |
| A-6 | 24 | 839 | 35 |
| A-7 | 22 | 409 | 19 |
| benznidazole | 870 | - | - |

<u>Example 9</u>

<u>Culture of the protozoa of Leishmaniasis</u>

This test was done using the protozoa of Leishmania donovani (MHOM/ET/67/L82 strain). The protozoa were subcultured using Syrian Golden hamster and an amastigote was obtained thereform. The amastigotes were cultured in SM medium supplemented with 10% of heat-treated fetal calf serum (pH 5.4) under $CO_2$ concentration of 5%, at 37°C.

<u>*In vitro* screening assay of growth-inhibiting effect for the protozoa of Leishmaniasis</u>

A test sample was prepared by dissolving the test compounds according to the present invention and a positive control drug (Miltefosine) in DMSO to a predetermined concentration. Culture medium containing a predetermined number of the protozoa was added into each well of 96-well culture plate, and the concentration of the amastigotes was measured by means of CASY cell analysis system (Dermany, Scharfe Co.). Test sample comprising the predetermined amount of the test compound, and DMSO without the test compound were added to the wells in duplicate.

After incubation of the culture plate for 72 hours in an incubator, a growth-inhibiting activity was assayed as

follows. Alamar Blue aqueous solution (10 μL) was added to each well followed by a further two-hour culture. The culture plate was then attached to a fluorescence micro titer plate reader (Spectramax Gemeni XS; U.S. Molecular device Co.), the intensity of fluorescence at 588nm was detected with excitation wave length of 536nm to calculate an infection ratio of the protozoa with respect to a group trated with the test sample and a control group. A growth-inhibiting ratio was then calculated from the resulting infection ratio in accordance with the following equation to determine 50% growth-inhibiting concentration ($EC_{50}$).

$$\text{Growth-inhibiting ratio (\%)} = [1-(b-a) / (c-a)] \times 100$$

a: Initial infection ratio;
b: Infection ratio of the test sample group; and
c: Infection ratio of the control group.

Determination of pharmaceutical effect for Leishmaniasis

A selective toxicity coefficient, which is used as an index for the selective toxicity for the protozoa of Leishmaniasis, was calculated by the following equation, and the pharmaceutical effect was determined.

A selective toxicity coefficient = (EC50 value of the test sample for rat L6 cell) /(EC50 value of the test sample for the protozoa of Leishmaniasis)

The values of EC50 for rat L6 cell and the protozoa of Leishmaniasis, and the selective toxicity of the compounds according to the present invention and the positive control drug are shown in TABLE 10. These results demonstrated that the compounds according to the present invention did show an excellent growth-inhibiting effect comparable to or more than Miltefosine known as a drug against Leishmaniasis, and a highly selective toxicity.

[TABLE 10]

| Compound | growth-inhibiting concentration (nM) | | Selective toxicity |
|---|---|---|---|
| | *Leishmania donovani* | Cytotoxicity L6 | |
| A-1 (60%purity) | 2810 | 3950 | 1.4 |
| A-3 | 887 | 8870 | 10 |
| A-6 | 23 | 839 | 36 |
| A-7 | 6.0 | 409 | 68 |
| Miltefosine | 280 | - | - |

Advantages of the invention

By using the compounds according to the present invention as the active ingredient, the excellent pharmaceutical composition for the treatment and/or prevention of the parasitic infection will be provided.

**Claims**

1. A pharmaceutical composition comprising the compound represented by the following general formula (1) as an active ingredient:

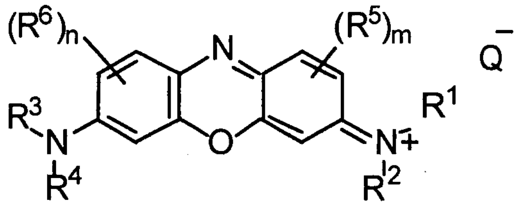

(1)

Wherein R1 and R2 may be the same or different, and independently represent alkyl, aryl, or heterocyclic group, which may optionally have a substituent of hydroxy, alkoxy, halogen, amino, cyano, sulfonic acid, carboxy, ester, amido, or nitro group, or may be condensed together to form a ring;
R3 and R4 may be the same or different, and independently represent alkyl, aryl, or heterocyclic group, which may optionally have a substituent of hydroxy, alkoxy, halogen, amino, cyano, sulfonic acid, carboxy, ester, amido, or nitro group, or may be condensed together to form a ring;
R5 and R6 may be the same or different, and independently represent halogen, alkyl, aryl, or heterocyclic, hydroxy, alkoxy, acyloxy, amino, cyano, sulfonic acid, carboxy, ester, amido, or nitro group, or may form cycloaliphatic ring, aromatic ring, hetero cycloaliphatic ring, or hetero aromatic ring;
m and n are independently represent an integer of 0 - 3; and
Q is a pharmaceutically acceptable anion.

**2.** A pharmaceutical composition according to Claim 1 wherein R1, R2, R3 and R4 are independently alkyl group having 1~6 carbon atoms.

**3.** A pharmaceutical composition according to Claim 1 or 2 wherein Q is halogen ion or perchlorate ion.

**4.** A pharmaceutical composition according to any one of Claims 1-3 wherein R1 and R2, or R3 and R4 are condensed together to form a ring.

**5.** A pharmaceutical composition according to Claim 4 wherein the ring is piperazine ring or morpholine ring.

**6.** A pharmaceutical composition comprising any one of the following compounds as an active ingredient:

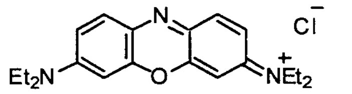
A-1

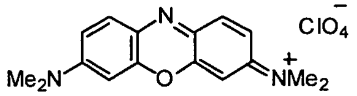
A-2

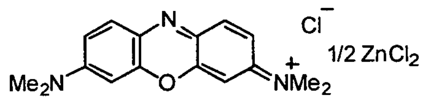
A-3

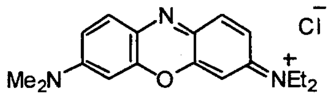
A-4

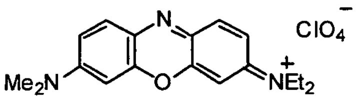
A-5

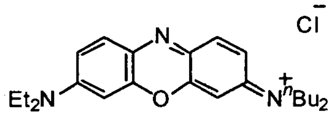
A-6

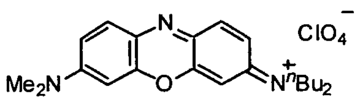
A-7

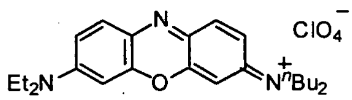
A-8

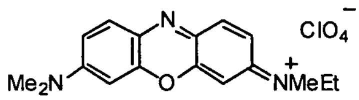
A-9

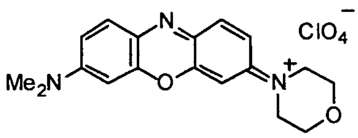
A-10

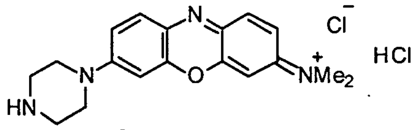
A-11

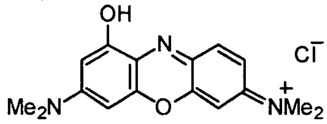
A-12

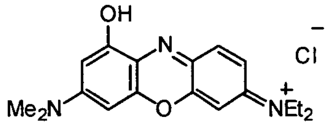
A-13

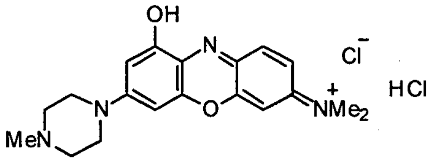
A-14

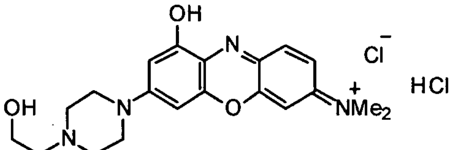
A-15

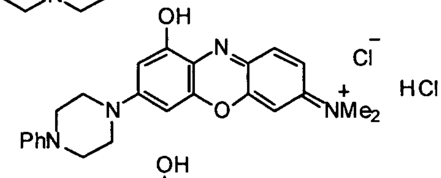
A-16

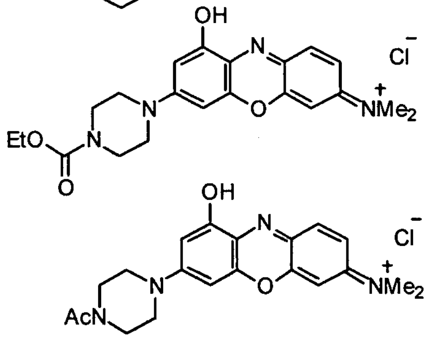
A-17

A-18

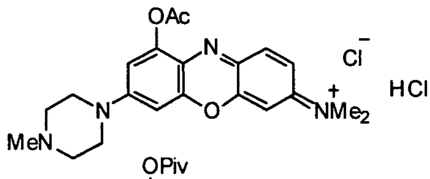
A-19

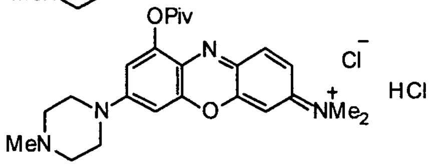
A-20

**7.** A pharmaceutical composition according to any one of Claims 1 - 6 for the treatment and/or prevention of parasitic infection by protozoa.

**8.** A pharmaceutical composition according to Claim 7 wherein the parasitic infection is malaria, leishmaniasis, African sleeping disease, Chagas' disease, toxoplasmosis, lymphofilariasis, babesiasis, or coccidiosis.

**9.** A pharmaceutical composition according to Claim 8 wherein the parasitic infection is malaria, leishmaniasis, African

sleeping disease or Chagas' disease.

**10.** A pharmaceutical composition according to Claim 9 wherein the parasitic infection is malaria.

**11.** A pharmaceutical composition according to any one of Claims 1 - 10 for the treatment and/or prevention of the parasitic infection by protozoa, comprising the compound represented by the general formula (1) in an amount of 1 mg ~ 10,000 mg.

**12.** A pharmaceutical composition according to any one of Claims 1 - 11 for the treatment and/or prevention of the parasitic infection by protozoa, wherein said composition is in a form of liquid, tablet, or pill or colloid.

**13.** A phenoxazinium compound represented by any one of the structures named as A-8, A-9, and A-11.

**INTERNATIONAL SEARCH REPORT**

International application No.
PCT/JP2006/302017

A. CLASSIFICATION OF SUBJECT MATTER
*A61K31/538*(2006.01), *A61P33/02*(2006.01), *A61P33/06*(2006.01), *C07D265/38*(2006.01)

According to International Patent Classification (IPC) or to both national classification and IPC

B. FIELDS SEARCHED

Minimum documentation searched (classification system followed by classification symbols)
A61K31/538, A61P33/02, A61P33/06, C07D265/38

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | VENNERSTORM, J. L. et al, Antimalarial Dyes Revisited: Xanthenes, Azines, Oxazines, and Thiazines, ANTIMICROBIAL AGENTS AND CHEMOTHERAPY, 1995, Vol39, No.12, pages 2671-2677 | 1-13 |
| A | JP 63-71190 A (HOECHST AG.), 31 March, 1988 (31.03.88), Full text & US 4861775 A | 1-13 |
| A | JP 2002-541144 A (DANA FARBER CANCER INST. INC.), 03 December, 2002 (03.12.02), Full text & WO 00/59884 A1 & EP 1154997 A1 | 1-13 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 March, 2006 (15.03.06) | 28 March, 2006 (28.03.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 0001968 W


### Non-patent literature cited in the description


- *Antimicrobial Agents and Chemotherapy,* 1995, vol. 39, 2671-2677
- **M.L. CROSSLEY.** *Journal of American Chemical Society,* 1952, vol. 74, 578-584
- **K. ANDREAS.** *European Journal of Organic Chemistry,* 1999, vol. 4, 923-930